# EUROPEAN PATENT APPLICATION

(11) **EP 2 752 663 A1**
(43) Date of publication of application: **09.07.2014**
(21) Application number: 14150335.9
(22) Date of filing: 07.01.2014
(51) Int. Cl.: G01N 33/50

(54) **Anti-inflammatory food product**

(30) Priority: 07.01.2013 US 201313735547
(71) Applicant: WhiteWave Services, Inc., Dallas TX 75204 (US)
(72) Inventor: Neal Allan, Bringe, Elizabeth, CO Colorado 80107-7120 (US)
(74) Representative: Grünecker, Kinkeldey, Stockmair & Schwanhäusser

(57) **Abstract**

According to some embodiments, a method selects one or more inflammatory markers to evaluate. The one or more inflammatory markers selected from the group consisting of iNOS, COX-2, TNF-alpha, NO, PGE₂, IL-6, and IL-1β. The method determines a first expression level and a second expression level of the one or more inflammatory markers. The first expression level is associated with a positive control sample comprising a type of human macrophages. The second expression level is associated with a test sample obtained by exposing a food product to a digest, the type of human macrophages, and a pro-inflammatory compound. The method performs a comparison between the first expression level and the second expression level and designates the food product according to an anti-inflammatory standard based on the comparison.

## Description

### TECHNICAL FIELD OF THE DISCLOSURE

This invention relates in general to food products and, more particularly, to an anti-inflammatory food product.

### BACKGROUND

Many people in the United States and other countries suffer from chronic inflammation. Chronic inflammation may be caused in part by disease, poor diet, and/or physical inactivity. Chronic inflammation may contribute to decline in mental and physical well-being, including lack of vitality, energy, and strength. People with chronic inflammation may benefit from eating fruits, vegetables, nuts, seeds, spices, soymilk, and other plant-based foods because plant-based foods tend to have anti-inflammatory components. These anti-inflammatory components may include polyphenols, such as the isoflavones in soybeans.

### SUMMARY

According to some embodiments, a method selects one or more inflammatory markers to evaluate. The one or more inflammatory markers selected from the group consisting of iNOS, COX-2, TNF-alpha, NO, PGE₂, IL-6, and IL-1β. The method determines a first expression level and a second expression level of the one or more inflammatory markers. The first expression level is associated with a positive control sample comprising a type of human macrophages. The second expression level is associated with a test sample obtained by exposing a food product to a digest, the type of human macrophages, and a pro-inflammatory compound. The method performs a comparison between the first expression level and the second expression level and designates the food product according to an anti-inflammatory standard based on the comparison.

Certain embodiments of the present disclosure may provide one or more technical advantages. As an example, in some embodiments, a food product is evaluated according to an anti-inflammatory standard. The food product may be a commercial food product, that is, a finished food product formulated by combining ingredients and/or processing them according to commercial processes. Combining and processing the ingredients may yield positive, negative, additive, and/or synergistic interactions that produce an overall anti-inflammatory or pro-inflammatory response. By evaluating the food product according to an anti-inflammatory standard, the formulation and processing decisions may be refined in order to develop food products with a sufficient level of anti-inflammatory response. These anti-inflammatory food products may help ameliorate symptoms for those consumers who suffer from chronic inflammation.

Other technical advantages of the present disclosure will be readily apparent to one skilled in the art from the following figures, descriptions, and claims. Moreover, while specific advantages have been enumerated above, various embodiments may include all, some, or none of the enumerated advantages.

### BRIEF DESCRIPTION OF THE DRAWINGS

For a more complete understanding of the present disclosure and its advantages, reference is now made to the following description, taken in conjunction with the accompanying drawing(s), in which:
FIGURE 1 illustrates an example of a method for designating a food product according to an anti-inflammatory standard;
FIGURE 2 illustrates an example of a soluble protein concentration of pepsin-pancreatin hydrolysates obtained from different commercially-available and prototype products: pre-process soymilk ("PPH"), yogurt ("YH"), strawberry-banana soymilk ("SBH"), mixed berry soymilk ("MXH"), orange juice ("OJH"), spiced pumpkin bread made with soybean okara ("PBRH"), tart cherry juice ("CJH"), vanilla soymilk ("SVMH"), and almond milk with cinnamon ("AMH");
FIGURES 3A-3B illustrate an example effect of the pepsin-pancreatin hydrolysates (400 µg/mL) obtained from the different commercially-available and prototype products on nitric oxide production (NO) (FIG. 3A) and iNOS expression (FIG. 3B) of LPS-stimulated RAW 264.7 macrophages (n = 3, p < 0.05);
FIGURES 4A-4B illustrate an example effect of the pepsin-pancreatin hydrolysates (400 µg/mL) obtained from the different commercially-available and prototype products on prostaglandin E₂ (PGE₂) secretion (FIG. 4A) and cyclooxygenase-2 (COX-2) expression (FIG. 4B) of LPS-stimulated RAW 264.7 macrophages (n = 3, p < 0.05);
FIGURES 5A-5C illustrate an example effect of the pepsin-pancreatin hydrolysates (400 µg/mL) obtained from the different commercially-available and prototype products on interleukin-6 (IL-6) (FIG. 5A), interleukin-1β (IL-1β) (FIG. 5B) and tumor necrosis factor-α (TNF-α) secretion (FIG. 5C) of LPS-stimulated. RAW 264.7 macrophages (n = 3, p < 0.05);
FIGURE 6 illustrates an example antioxidant capacity (ORAC, µM Trolox equivalent) of the pepsin-pancreatin hydrolysates (400 µg/mL) from the different commercially-available and prototype products (n = 3, p< 0.05); and
FIGURE 7 illustrates an example correlation (Pearson r) between anti-inflammatory markers and antioxidant capacity of different commercially-available and prototype soybean products.

### DETAILED DESCRIPTION

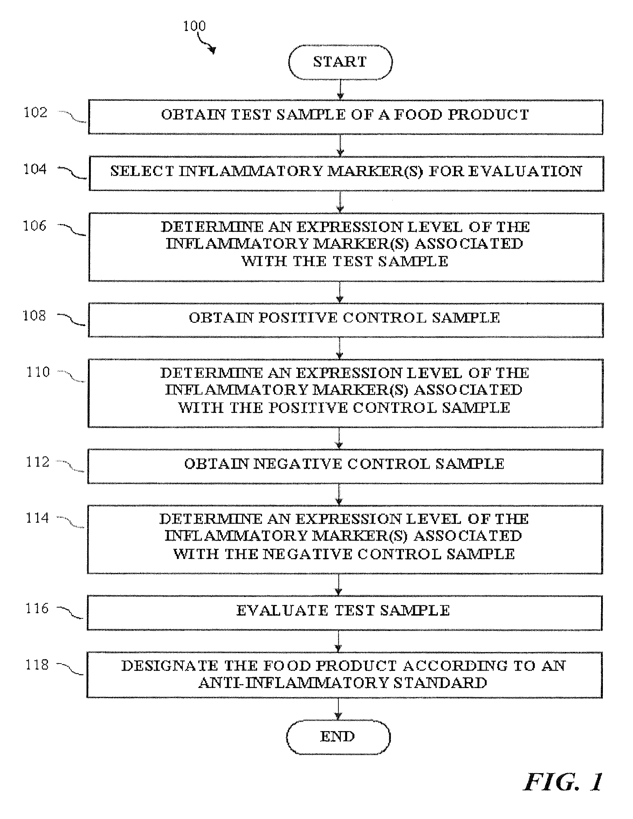

Embodiments of the present invention and its advantages are best understood by referring to FIGURES 1 to 7 of the drawings, like numerals being used for like and corresponding parts of the various drawings.

Many people in the United States and other countries suffer from chronic inflammation. Chronic inflammation may be caused in part by disease, poor diet, and/or physical inactivity. Chronic inflammation may contribute to decline in mental and physical well-being, including lack of vitality, energy, and strength. People with chronic inflammation may benefit from eating fruits, vegetables, nuts, seeds, spices, soymilk, and other plant-based foods because plant-based foods tend to have anti-inflammatory components. These anti-inflammatory components may include polyphenols, such as the isoflavones in soybeans.

Certain plant-based foods may have a better anti-inflammatory response than others. In addition, the anti-inflammatory response associated with a particular plant-based ingredient may change as the ingredient is processed to make a commercial food product. As an example, combining a plant-based ingredient, such as soymilk, with other plant-based ingredients, such as fruit-based ingredients and/or spices, may change the anti-inflammatory response of the soy and fruit/spices. In some cases, the combination of ingredients may yield an overall positive, additive, and/or synergistic interaction that produces an improvement in the overall anti-inflammatory response. In other cases, the combination of ingredients may yield an overall negative interaction that produces a reduction in the overall anti-inflammatory response or an increase in pro-inflammatory response. Similarly, commercial processing may affect the anti-inflammatory response of the plant-based ingredients. As an example, changes to the pasteurization time and/or pasteurization temperature may either improve or worsen the anti-inflammatory response.

Embodiments of the present disclosure may allow for evaluating various food products as a whole, that is, after all of the ingredients have been added and the processing steps have been completed. The evaluation may be used to designate food products with a sufficient level of anti-inflammatory response. These anti-inflammatory food products may help ameliorate symptoms for those consumers who suffer from chronic inflammation.

FIGURE 1 illustrates an example of a method 100 for designating a food product according to an anti-inflammatory standard. Method 100 generally comprises determining an expression level of one or more inflammatory markers associated with a test sample of a food product and designating the food product according to an anti-inflammatory standard based accordingly.

A test sample of a food product is obtained at step 102. The test sample may comprise a hydrolysate of any suitable food product, such as non-dairy milk (soymilk, flavored soymilk, fortified soymilk, almond milk, etc.), juice (e.g., cherry juice, orange juice, etc.), bread (e.g., pumpkin spiced bread), yogurt (e.g., soy yogurt), or any other food or beverage. In some embodiments, the food product is a whole food product for which all of the ingredients have been added and pasteurization, homogenization, and/or other processing steps have been completed.

The test sample may be obtained by exposing the food product to one or more digests, a type of human macrophages, and a pro-inflammatory compound. In some embodiments, the food product is first exposed to a combination of the digest(s) and the human macrophages for approximately 1-3 hours. As an example, the food product may be exposed to the macrophages, such as RAW 264.7 macrophages, and a first digest, such as pepsin, for approximately 1.5 hours followed by a second digest, such as pancreatin, for approximately 1.5 hours. The resulting mixture may be filtered to remove salt and then exposed to the human macrophages and the pro-inflammatory compound for approximately 20-30 hours (e.g., 24 hours). The pro-inflammatory compound may comprise lipopolysaccharide or any suitable compound for simulating an unhealthy/inflammatory condition.

At step 104, one or more inflammatory markers are selected for evaluation. Inflammatory markers that may be well-suited to evaluating the anti-inflammatory response of a whole food product include one or more of: inducible nitric oxide synthase (iNOS), cyclooxygenase-2 (COX-2), tumor necrosis factor-α (TNF-alpha), nitric oxide (NO), prostaglandin E₂ (PGE2), interleukin-6 (IL-6), and interleukin 1β (IL-1β). In some embodiments, each of the preceding markers may be selected. In some alternative embodiments, at least iNOS, COX-2, TNF-alpha, and PGE₂ may be selected as particularly well-suited to discriminating the anti-inflammatory response of a whole food product, such as a non-dairy milk beverage (e.g., a plant-based beverage, such as a soymilk, almond milk, coconut milk, rice milk, etc.). However, any suitable combination of one or more inflammatory markers may be selected.

An expression level of the selected inflammatory markers associated with the test sample may be determined at step 106. An example of expression levels of the inflammatory markers may be found in Table 1, discussed further below.

A positive control sample may be obtained at step 108. The positive control sample may represent a healthy condition. Thus, the positive control sample may be characterized by the absence of the pro-inflammatory compound (e.g., the positive control sample is not exposed to lipopolysaccharide). The positive control sample may comprise the same type of human macrophages that were used to obtain the test sample, such as RAW 264.7 macrophages.

At step 110, an expression level of the selected inflammatory markers associated with the positive control sample may be determined. An example of expression levels of the inflammatory markers may be found in Table 1 below.

At step 112, a negative control sample may be obtained. The negative control sample may represent the unhealthy/inflamed condition. Thus, the positive control sample may comprise the same type of human macrophages, such as RAW 264.7 macrophages, exposed to the pro-inflammatory compound (e.g., lipopolysaccharide). In some embodiments, the human macrophages of the negative control sample may be exposed to the pro-inflammatory compound for approximately the same amount of time that the human macrophages of the test sample are exposed to the pro-inflammatory compound, such as 20-30 hours (e.g., 24 hours).

An expression level of the selected inflammatory markers associated with the negative control sample may be determined at step 114. An example of expression levels of the inflammatory markers may be found in Table 1 below.

At step 116, each of the expression level of each of the selected inflammatory markers associated with the test sample may be evaluated. The evaluation may determine whether the expression level of each inflammatory marker indicates an anti-inflammatory response, a neutral response, or a pro-inflammatory response.

In some embodiments, this determination may be made for each of the selected inflammatory markers by comparing the expression level associated with the test sample to the expression level associated with one or both of the control samples. As an example, an inflammatory marker may be determined to indicate an anti-inflammatory response if its expression level in the test sample is sufficiently reduced as compared to its expression level in the negative control sample (unhealthy condition). A predetermined threshold may be used to indicate sufficient reduction in the expression level. In some embodiments, the predetermined threshold may indicate at least a 15% reduction in each of the selected inflammatory markers. Examples of predetermined thresholds may be at least a 62% reduction in COX-2, at least a 40% reduction in TNF-alpha, at least a 19% reduction in NO, at least a 66% reduction level in PGE₂, at least a 24% reduction in IL-6, and/or at least a 25% reduction in IL-1β.

As another example, an inflammatory marker may be determined to indicate an anti-inflammatory response if its expression level in the test sample is statistically not significantly different than its expression level in the positive control sample (healthy condition). In some embodiments, the expression level may be determined to be statistically not significantly different if the p value is less than 0.05. In some embodiments, the statistical analysis may express the results as the mean +/- standard deviation (SD). In some embodiments, statistical comparisons may be made by one-way analysis of variance (ANOVA), followed by Duncan's multiple-comparison test.

As another example, an inflammatory marker may be determined to be anti-inflammatory based on a scoring system where its expression level in the positive control sample corresponds to a good score (healthy condition), its expression level in the negative control sample corresponds to a bad score (unhealthy condition), and its expression level in the test sample is scored relative to the good score and the bad score. Accordingly, an expression level closer to the negative control sample would receive a better score than an expression level closer to the positive control sample.

At step 118, the food product is designated according to an anti-inflammatory standard. In some embodiments, the anti-inflammatory standard comprises a healthy bioactivity index. Examples of indexes are described with respect to Examples A - J below. In general, the index may indicate whether to designate the food product as anti-inflammatory, neutral, or pro-inflammatory. The index may be based on a net score. The net score may be calculated by adding together the scores associated with each of the selected inflammatory markers. In some embodiments, designating the food product may include identifying a neutral or pro-inflammatory developmental product as requiring further development or identifying an anti-inflammatory developmental product as approved for launch or clinical trial In some embodiments, designating the food product may include labeling the product as anti-inflammatory, as having a healthy bioactivity level, or similar designation on a package or advertisement associated with the food product

Steps 102-118 may optionally be repeated for any suitable number of food products Evaluating and comparing multiple food products may facilitate making decisions about how well a particular food product performs well The method then ends

Method 100 was used to evaluate a number of example food products Table 1 below describes the results of the tests. In Table 1, Example A tested strawberry banana soymilk, Example B tested mixed berry soymilk, Example C tested vanilla soymilk, Example D tested yogurt, Example E tested tart cherry juice, Example F tested spiced almond milk, Example G tested orange juice, Example H tested pumpkin spiced bread with okara, Example I tested test soymilk, and Example J tested test soymilk with DHA

The following measurements are based on the mean plus or minus the standard error of the mean (SEM) Results were generated using 400 micrograms per milliliter (µg/mL) of each example hydrolysate The expression levels of iNOS and COX-2 are given as relative expression levels The expression levels of TNF-alpha, PGE2, IL-6, and IL-1B are given in nanograms per milliliter (ng/mL). The expression level of NO is given in micromolar (µM) units To standardize the results to the same amount of protein, the expression levels correspond to values of intensity in a western membrane for a specific protein in relation with the values of intensity for a standard level of a control protein (actin) added to each sample

**Table 1**

| | iNOS | COX-2 | TNF-alpha | NO | PGE₂ | IL-6 | IL-1β |
|---|---|---|---|---|---|---|---|
| Negative Control (without LPS) | 0.37 ± 0.08 | 0.17 ± 0.10 | 244.8 ± 48.6 | -7.4 ± 0.8 | 0.16 ± 0.1 | -1.05 ± 0.2 | 0.48 ± 0.1 |
| Example A | 0.33 ± 0.17 | 0.38 ± 0.16 | 568.0 ± 50.2 | 31.0 ± 2.8 | 10.40 ± 1.6 | 46.17 ± 6.6 | 15.65 ± 0.7 |
| Example B | 0.35 ± 0.05 | 0.30 ± 0.09 | 545.0 ± 47.5 | 35.9 ± 2.3 | 12.46 ± 2.4 | 42.29 ± 7.6 | 12.85 ± 1.4 |
| Example C | 0.12 ± 0.04 | 0.59 ± 0.02 | 478.9 ± 33.3 | 30.7 ± 3.8 | 44.11 ± 2.2 | 31.68 ± 1.1 | 16.65 ± 1.6 |
| Example D | 0.85 ± 0.17 | 0.65 ± 0.08 | 572.9 ± 42.7 | 34.7 ± 2.2 | 19.50 ± 4.0 | 66.61 ± 7.6 | 15.65 ± 1.3 |
| Example E | 0.62 ± 0.20 | 0.71 ± 0.05 | 454.2 ± 37.6 | 39.3 ± 0.4 | 29.47 ± 1.7 | 24.74 ± 1.6 | 16.05 ± 1.5 |
| Example F | 0.68 ± 0.21 | 0.54 ± 0.01 | nd | 43.6 ± 0.2 | nd | nd | 15.25 ± 1.5 |
| Example G | 1.00 ± 0.00 | 1.19 ± 0.15 | 763 ± 26.5 | 35.4 ± 4.1 | 20.41 ± 3.3 | 35.01 ± 0.3 | 25.15 ± 2.7 |
| Example H | 0.45 ± 0.05 | 1.19 ± 0.15 | 583.5 ± 25.3 | 42.5 ± 0.2 | 37.33 ± 3.6 | 54.19 ± 0.9 | 20.05 ± 2.3 |
| Example I | 1.17 ± 0.14 | 2.07 ± 0.02 | 679.0 ± 21.7 | 45.8 ± 0.2 | 68.47 ± 4.7 | 49.82 ± 1.0 | 19.25 ± 2.1 |
| Example J | 0.71 ± 0.06 | 2.97 ± 0.47 | 649.8 ± 17.6 | 45.1 ± 0.6 | 41.66 ± 5.0 | 51.11 ± 1.0 | 20.05 ±2.7 |
| Negative control (with LPS) | 1.00 + 0.00 | 1.00 ± 0.00 | 953.7 ± 112.4 | 43.0 ± 0.3 | 30.42 ± 1.5 | 43.51 ± 1.6 | 21.25 ± 1.2 |

The test results may be evaluated according to an anti-inflammatory standard described by a Healthy Bioactivity Index. A Healthy Bioactivity Index describes any suitable classification and/or scoring method that allows for distinguishing test samples with good anti-inflammatory response from test samples with poor anti-inflammatory response As an example, the test results shown in Table 1 were evaluated according to the example anti-inflammatory standards of Healthy Bioactivity Index A and Healthy Bioactivity Index B In Index A and Index B, each of the selected inflammatory markers was coded as red, blue, black, gold, or purple as shown in more detail with respect to Examples A-J below

Red indicated that the expression level of the test sample was not significantly different from the positive control sample (healthy condition) For example, the expression level may be not significantly different if the p value is less than 0.05 with respect to the positive control sample Inflammatory markers coded as red were given a good score, here, 3 points.

Blue and black color codes refer to expression levels that statistically fall somewhere in between the positive control sample and the negative control sample Thus, blue and black codes indicate the expression level was not statistically the same as either the positive control or the negative control (the p value with respect to the positive control sample was greater than 0.05 and the p value with respect to the negative control sample was greater than 0.05). Of these samples, blue indicated that the expression level of the test sample was among the best set of samples not statistically different from each other. Inflammatory markers coded as blue were given a somewhat good score, here, 1 point. Black indicated that the expression level of the test sample did not qualify for one of the other color codes. Inflammatory markers coded as black were given a neutral score, here, 0.

Gold indicated that the expression level of the test sample was not significantly different from the negative control sample (unhealthy condition). For example, the expression level may be not significantly different if the p value is less than 0.05 with respect to the negative control sample. Inflammatory markers coded as gold were given a somewhat bad score, here, -2 points.

Purple indicated that the expression level of the test sample was significantly worse than the negative control sample. Inflammatory markers coded as purple were given a bad score, here, -4 points.

In Index A, iNOS, COX-2, TNF-α, NO, PGE₂, IL-6, and IL-1β are selected as the inflammatory markers. The individual scores of each selected marker were added together to yield a net score used to designate the food product. According to Index A, an anti-inflammatory designation corresponds to a net score greater than or equal to 5, a neutral designation corresponds to a net score between -12 and 4, and a pro-inflammatory designation corresponds to a net score less than or equal to -13.

In Index B, iNOS, COX-2, TNF-α, and PGE₂ are selected as the inflammatory markers. The individual scores of each selected marker were added together to yield a net score used to designate the food product. According to Index B, an anti-inflammatory designation corresponds to a net score greater than or equal to 5, a neutral designation corresponds to a net score between -9 and 4, and a pro-inflammatory designation corresponds to a net score less than or equal to -10.

Thus, in some embodiments, a food or beverage, such as a plant-based beverage, may be designated as anti-inflammatory if it comprises at least 1 gram of protein per 8 ounce serving and has a bioactivity index greater than or equal to 5 according to Index A and/or Index B disclosed herein. In some embodiments, certain soymilk combined with one or more of a vegetable ingredient (e.g., vegetable juice, vegetable puree, vegetable powder, vegetable extract), a fruit ingredient (e.g, juice, puree, powder, extract, etc. from one or more of stawberries, bananas, raspberries, blackberries, cherries, oranges, or other suitable fruits), a plant-based protein ingredient, seed(s), herb(s), spice(s), and a yogurt culture(s), and processed according to commercial processes (e g , pasteurized and/or homogenized) may meet this anti-inflammatory standard

### Example A - Strawberry Banana Soymilk

Example A comprises a soymilk with strawberry banana flavor Table 2 describes the nutritional composition of Example A

**Table 2**

| **Strawberry Banana Soymilk** | |
|---|---|
| Protein | 5 g/240-mL |
| Fat | 1.5 g/240-mL |
| Sugar | 19 g/240-mL |
| Fiber | 2 g/240-mL |
| Vitamin A | 0% daily value |
| Vitamm C | 100% daily value |
| Vitamin D | 25% daily value |
| Vitamin B2 | -- % daily value |
| Vitamin B12 | -- % daily value |
| Calcium | 20% daily value |
| Magnesium | -- % daily value |
| Iron | 4% daily value |
| Phosphorous | -- % daily value |
| Folate | 6% daily value |

Table 3 compares Example A to the positive and positive control samples in order to evaluate the food product according to the anti-inflammatory standard defined by Index A and the anti-inflammatory standard defined by Index B

**Table 3**

| | **Positive control** | **Example A** | **Negative control** | **Code** | **Index A** | **Index B** |
|---|---|---|---|---|---|---|
| iNOS | 0.37 ± 0.08 | 0.33 ± 0.17 | 1.00 ± 0.00 | Red | 3 | 3 |
| COX-2 | 0.17 ± 0.10 | 0.38 ± 0.16 | 1.00 ± 0.00 | Red | 3 | 3 |
| TNF-α | 244.8 ± 48.6 | 568.0 ± 50.2 | 953.7 ± 112.4 | Red | 3 | 3 |
| NO | -7.4 ± 0.8 | 31.0 ± 2.8 | 43.0 ± 0.3 | Blue | 1 | n/a |
| PGE2 | 0.16 ± 0.1 | 10.40 ± 1.6 | 30.42 ± 1.5 | Red | 3 | 3 |
| IL-6 | -1.05 ± 0.2 | 46.17 ± 6.6 | 43.51 ± 1.6 | Gold | -2 | n/a |
| IL-1β | 0.48 ± 0.1 | 15.65 ± 0.7 | 21.25 ± 1.2 | Blue | 1 | n/a |
| | | | | Total | 12 | 12 |

According to Index A, Example A was designated as anti-inflammatory According to Index B, Example A was designated as anti-inflammatory

### Example B - Mixed Berry Soymilk

Example B comprises a soymilk with mixed berry flavor Table 4 describes the nutritional composition of Example B.

**Table 4**

| **Mixed Berry Soymilk** | |
|---|---|
| Protein | 5 g/240-mL |
| Fat | 1 5 g/240-mL |
| Sugar | 19 g/240-mL |
| Fiber | 2 g/240-mL |
| Vitamin A | 0% daily value |
| Vitamin C | 100% daily value |
| Vitamin D | 25% daily value |
| Vitamin B2 | -- % daily value |
| Vitamin B12 | -- % daily value |
| Calcium | 20% daily value |
| Magnesium | -- % daily value |
| Iron | 4% daily value |
| Phosphorous | -- % daily value |
| Folate | 6% daily value |

Table 5 compares Example B to the positive and positive control samples in order to evaluate the food product according to the anti-inflammatory standard defined by Index A and the anti-inflammatory standard defined by Index B.

**Table 5**

| | **Positive control** | **Example B** | **Negative control** | **Code** | **Index A** | **Index B** |
|---|---|---|---|---|---|---|
| iNOS | 0.37 ± 0.08 | 0.35 ± 0.05 | 1.00 ± 0.00 | Red | 3 | 3 |
| COX-2 | 0.17 ± 0.10 | 0.30 ± 0.09 | 1.00 ± 0.00 | Red | 3 | 3 |
| TNF-α | 244.8 ± 48.6 | 545.0 ± 47 5 | 953.7 ± 112.4 | Red | 3 | 3 |
| NO | -7.4 ± 0.8 | 35.9 ± 2.3 | 43.0 ± 0.3 | Black | 0 | n/a |
| PGE2 | 0.16 ± 0.1 | 12.46 ± 24 | 30.42 ± 1.5 | Blue | 1 | 1 |
| IL-6 | -1.05 ± 0 2 | 42.29 ± 7.6 | 43.51 ± 1.6 | Gold | -2 | n/a |
| IL-1β | 0.48 ± 0.1 | 12.85 ± 1.4 | 21.25 ± 1.2 | Blue | 1 | n/a |
| | | | | Total | 9 | 10 |

According to Index A, Example B was designated as anti-inflammatory According to Index B, Example B was designated as anti-inflammatory

### Example C - Vanilla Soymilk

Example C comprises a soymilk with vanilla flavor Table 6 describes the nutritional composition of Example C

**Table 6**

| **Vanilla Soymilk** | |
|---|---|
| Protein | 6 g/240-mL |
| Fat | 3.5 g/240-mL |
| Sugar | 8 g/240-mL |
| Fiber | 1 g/240-mL |
| Vitamin A | 10% daily value |
| Vitamin C | 0% daily value |
| Vitamm D | 30% daily value |
| Vitamin B2 | 25% daily value |
| Vitamin B12 | 50% daily value |
| Calcium | 45% daily value |
| Magnesium | 10% daily value |
| Iron | 6% daily value |
| Phosphorous | 8% daily value |
| Folate | 10% daily value |

Table 7 compares Example C to the positive and positive control samples in order to evaluate the food product according to the anti-inflammatory standard defined by Index A and the anti-inflammatory standard defined by Index B

**Table 7**

| | **Positive control** | **Example C** | **Negative control** | **Code** | **Index A** | **Index B** |
|---|---|---|---|---|---|---|
| iNOS | 0.37 ± 0.08 | 0.12 ± 0.04 | 1.00 ± 0.00 | Red | 3 | 3 |
| COX-2 | 0.17 ± 0.10 | 0.59 ± 0.02 | 1.00 ± 0.00 | Black | 0 | 0 |
| TNF-α | 244.8 ± 48.6 | 478.9 ± 33.3 | 953.7 ± 112.4 | Red | 3 | 3 |
| NO | -7.4 ± 0.8 | 30.7 ± 3.8 | 43.0 ± 0.3 | Blue | 1 | n/a |
| PGE2 | 0.16 ± 0.1 | 44.11 ± 2.2 | 30.42 ± 1.5 | Black | 0 | 0 |
| IL-6 | -1.05 ± 0.2 | 31.68 ± 1.1 | 43.51 ± 1.6 | Blue | 1 | n/a |
| IL-1β | 0.48 ± 0.1 | 16.65 ± 1.6 | 21.25 ± 1.2 | Gold | -2 | n/a |
| | | | | Total | 6 | 6 |

According to Index A, Example C was designated as anti-inflammatory According to Index B, Example C was designated as anti-inflammatory

### Example D - Yogurt

Example D comprises a yogurt. Table 8 describes the nutritional composition of Example D

**Table 8**

| **Yogurt** | |
|---|---|
| Protein | 6 g/240-mL |
| Fat | 3 5 g/240-mL |
| Sugar | 19 g/240-mL |
| Fiber | 2 g/240-mL |
| Vitamin A | 0% daily value |
| Vitamin C | 0% daily value |
| Vitamin D | 20% daily value |
| Vitamin B2 | -- % daily value |
| Vilamin B12 | -- % daily value |
| Calcium | 20% daily value |
| Magnesium | -- % daily value |
| Iron | 4% daily value |
| Phosphorous | 15% daily value |
| Folate | -- % daily value |

Table 9 compares Example D to the positive and positive control samples in order to evaluate the food product according to the anti-inflammatory standard defined by Index A and the anti-inflammatory standard defined by Index B

**Table 9**

| | **Positive control** | **Example D** | **Negative control** | **Code** | **Index A** | **Index B** |
|---|---|---|---|---|---|---|
| iNOS | 0.37 ± 0.08 | 0.85 ± 0.17 | 1.00 ± 0.00 | Gold | -2 | -2 |
| COX-2 | 0.17 ± 0.10 | 0.65 ± 0.08 | 1.00 ± 0.00 | Gold | -2 | -2 |
| TNF-α | 244.8 ± 48.6 | 572.9 ± 42.7 | 953.7 ± 112.4 | Red | 3 | 3 |
| NO | -7.4 ± 0.8 | 34.7 ± 2.2 | 43.0 ± 0.3 | Blue | 1 | n/a |
| PGE2 | 0.16 ± 0.1 | 19.50 ± 4.0 | 30.42 ± 1.5 | Blue | 1 | 1 |
| IL-6 | -1.05 ± 0.2 | 66.61 ± 7.6 | 43.51 ± 1.6 | Black | 0 | n/a |
| IL-1β | 0.48 ± 0.1 | 15.65 ± 1.3 | 21.25 ± 1.2 | Blue | 1 | n/a |
| | | | | Total | 2 | 0 |

According to Index A, Example D was designated as neutral According to Index B, Example D was designated as neutral

### Example E - Tart Cherry Juice

Example E comprises cherry juice. Table 10 describes the nutritional composition of Example E

**Table 10**

| **Tart Cherry Juice** | |
|---|---|
| Protein | 1 g/240-mL |
| Fat | 0 g/240-mL |
| Sugar | 28 g/240-mL |
| Fiber | 0 g/240-mL |
| Vitamin A | 6% daily value |
| Vitamin C | 0% daily value |
| Vitamin D | -- % daily value |
| Vitamin B2 | -- % daily value |
| Vitamin B12 | -- % daily value |
| Calcium | 2% daily value |
| Magnesium | % daily value |
| Iron | 4% daily value |
| Phosphorous | -- % daily value |
| Folate | -- % daily value |

Table 11 compares Example E to the positive and positive control samples in order to evaluate the food product according to the anti-inflammatory standard defined by Index A and the anti-inflammatory standard defined by Index B.

**Table 11**

| | **Positive control** | **Example E** | **Negative control** | **Code** | **Index A** | **Index B** |
|---|---|---|---|---|---|---|
| iNOS | 0.37 ± 0.08 | 0.62 ± 0.20 | 1.00 ± 0.00 | Black | 0 | 0 |
| COX-2 | 0.17 ± 0.10 | 0.71 ± 0.05 | 1.00 ± 0.00 | Gold | -2 | -2 |
| TNF-α | 244.8 ± 48.6 | 454.2 ± 37 6 | 953.7 ± 112.4 | Red | 3 | 3 |
| NO | -7.4 ± 0.8 | 39.3 ± 0.4 | 43.0 ± 0.3 | Gold | -2 | n/a |
| PGE2 | 0.16 ± 0.1 | 29.47 ± 1.7 | 30.42 ± 1.5 | Gold | -2 | -2 |
| IL-6 | -1.05 ± 0.2 | 24.74 ± 1.6 | 43.51 ± 1.6 | Blue | 1 | n/a |
| IL-1β | 0.48 ± 0.1 | 16.05 ± 1.5 | 21.25 ± 1.2 | Blue | 1 | n/a |
| | | | | Total | -1 | -1 |

According to Index A, Example E was designated as neutral According to Index B, Example E was designated as neutral

### Example F - Spiced Almond milk

Example F comprises a spiced almond milk. Table 12 compares Example F to the positive and positive control samples in order to evaluate the food product according to the anti-mflammatory standard defined by Index A and the anti-inflammatory standard defined by Index B

**Table 12**

| | **Positive control** | **Example F** | **Negative control** | **Code** | **Index A** | **Index B** |
|---|---|---|---|---|---|---|
| iNOS | 0.37 ± 0.08 | 0.68 ± 0.21 | 1.00 ± 0.00 | Black | 0 | 0 |
| COX-2 | 0.17 ± 0.10 | 0.54 ± 0.01 | 1.00 ± 0.00 | Red | 3 | 3 |
| TNF-α | 244.8 ± 48.6 | no data | 953.7 ± 112.4 | n.d | n.d | n.d. |
| NO | -7.4 ± 0.8 | 43.6 ± 0.2 | 43.0 ± 0.3 | Gold | -2 | n/a |
| PGE2 | 0.16 ± 0.1 | no data | 30.42 ± 1.5 | n.d | n.d. | n.d. |
| IL-6 | -1.05 ± 0.2 | no data | 43.51 ± 1.6 | nd | n.d. | n/a |
| IL-1β | 0.48 ± 0.1 | 15.25 ± 1.5 | 21.25 ± 1.2 | Blue | 1 | n/a |
| | | | | Total | 2* | 3* |

| | | | | | | |
|---|---|---|---|---|---|---|
| *Example F provisionally scored in the neutral range, however, data was not collected for the TNF-alpha, PGE2, or IL-6 markers. | | | | | | |

### Example G - Orange Juice

Example G comprises an orange juice. Table 13 describes the nutritional composition of Example G.

**Table 13**

| **Orange Juice** | |
|---|---|
| Protein | 1 g/240-mL |
| Fat | 0 g/240-mL |
| Sugar | 23 g/240-mL |
| Fiber | 0 g/240-mL |
| Vitamin A | 0% daily value |
| Vitamin C | 100% daily value |
| Vitamin D | -- % daily value |
| Vitamin B2 | -- % daily value |
| Vitamin B12 | -- % daily value |
| Calcium | 2% daily value |
| Magnesium | -- % daily value |
| Iron | 0% daily value |
| Phosphorous | -- % daily value |
| Folate | -- % daily value |

Table 14 compares Example G to the positive and positive control samples in order to evaluate the food product according to the anti-inflammatory standard defined by Index A and the anti-inflammatory standard defined by Index B

**Table 14**

| | **Positive control** | **Example G** | **Negative control** | **Code** | **Index A** | **Index B** |
|---|---|---|---|---|---|---|
| iNOS | 0.37 ± 0.08 | 1.00 ± 0.00 | 1.00 ± 0.00 | Gold | -2 | -2 |
| COX-2 | 0.17 ± 0.10 | 1.19 ± 0.15 | 1.00 ± 0.00 | Gold | -2 | -2 |
| TNF-α | 244.8 ± 48.6 | 763 ± 26.5 | 953.7 ± 112.4 | Gold | -2 | -2 |
| NO | -7.4 ± 0.8 | 35.4 ± 4.1 | 43.0 ± 0.3 | Black | 0 | n/a |
| PGE2 | 0.16 ± 0.1 | 20.41 ± 3.3 | 30.42 ± 1.5 | Blue | 1 | 0 |
| IL-6 | -1.05 ± 0.2 | 35.01 ± 0.3 | 43.51 ± 1.6 | Blue | 1 | n/a |
| IL-1β | 0.48 ± 0.1 | 25.15 ± 2.7 | 21.25 ± 1.2 | Gold | -2 | n/a |
| | | | | Total | -6 | 6 |

According to Index A, Example G was designated as neutral According to Index B, Example G was designated as neutral.

### Example H - Pumpkin spiced bread with okara

Example H comprises pumpkin spiced bread with okara Table 15 compares Example H to the positive and positive control samples in order to evaluate the food product according to the anti-inflammatory standard defined by Index A and the anti inflammatory standard defined by Index B

**Table 15**

| | **Positive control** | **Example H** | **Negative control** | **Code** | **Index A** | **Index B** |
|---|---|---|---|---|---|---|
| iNOS | 0.37 ± 0.08 | 0.45 ± 0.05 | 1.00 ± 0.00 | Black | 0 | 0 |
| COX-2 | 0.17 ± 0.10 | 1.19 ± 0.15 | 1.00 ± 0.00 | Gold | -2 | -2 |
| TNF-α | 244.8 ± 48.6 | 583.5 ± 25.3 | 953.7 ± 112.4 | Black | 0 | 0 |
| NO | -7.4 ± 0.8 | 42.5 ± 0.2 | 43.0 ± 0.3 | Gold | -2 | n/a |
| PGE2 | 0.16 ± 0.1 | 37.33 ± 3.6 | 30.42 ± 1.5 | Gold | -2 | -2 |
| IL-6 | -1.05 ± 0.2 | 54.19 ± 0.9 | 43.51 ± 1.6 | Purple | -4 | n/a |
| IL-1β | 0.48 ± 0.1 | 20.05 ± 2.3 | 21.25 ± 1.2 | Gold | -2 | n/a |
| | | | | Total | -12 | -4 |

According to Index A, Example H was designated as neutral According to Index B, Example H was designated as neutral

### Example I- Test soymilk

Example I comprises a experimental soymilk. Table 16 describes the nutritional composition of Example I

**Table 16**

| **Test soymilk** | |
|---|---|
| Protein | 7 g/240-mL |
| Fat | 4 g/240-mL |
| Sugar | 1 g/240-mL |
| Fiber | 2 g/240-mL |
| Vitamin A | 10% daily value |
| Vitamin C | 0% daily value |
| Vitamin D | 30% daily value |
| Vitamin B2 | 30% daily value |
| Vitamin B12 | 50% daily value |
| Calcium | 30% daily value |
| Magnesium | 10% daily value |
| Iron | 6% daily value |
| Phosphorous | 10% daily value |
| Folate | 15% daily value |

Table 17 compares Example I to the positive and positive control samples in order to evaluate the food product according to the anti-inflammatory standard defined by Index A and the anti-inflammatory standard defined by Index B

**Table 17**

| | **Positive control** | **Example I** | **Negative control** | **Code** | **Index A** | **Index B** |
|---|---|---|---|---|---|---|
| iNOS | 0.37 ± 0.08 | 1.17 ± 0.14 | 1.00 ± 0.00 | Gold | -2 | -2 |
| COX-2 | 0.17 ± 0.10 | 2.07 ± 0.02 | 1.00 ± 0.00 | Purple | -4 | 4 |
| TNF-α | 244.8 ± 48.6 | 679.0 ± 21.7 | 953.7 ± 112.4 | Gold | -2 | -2 |
| NO | -7.4 ± 0.8 | 45.8 ± 0.2 | 43.0 ± 0.3 | Gold | -2 | n/a |
| PGE2 | 0.16 ± 0.1 | 68.47 ± 4.7 | 30.42 ± 1.5 | Gold | -2 | -2 |
| IL-6 | -1.05 ± 0.2 | 49.82 ± 1.0 | 43.51 ± 1.6 | Gold | 2 | n/a |
| IL-1β | 0.48 ± 0.1 | 19.25 ± 2.1 | 21.25 ± 1.2 | Gold | -2 | n/a |
| | | | | Total | -16 | -10 |

According to Index A, Example I was designated as pro-inflammatory According to Index B, Example I was designated as pro-inflammatory.

### Example J - Test soymilk with DHA

Example J compnses an experimental soymilk with DHA and flax seed oil. Table 18 describes the nutritional composition of Example J

**Table 18**

| **Test soymilk with DHA** | |
|---|---|
| Protein | 7 g/240 mL |
| Fat | 5 g/240-mL |
| Sugar | 1 g/240-mL |
| Fiber | 2 g/240-mL |
| Vitamin A | 10% daily value |
| Vitamin C | 0% daily value |
| Vitamin D | 30% daily value |
| Vitamin B2 | 30% daily value |
| Vitamin B12 | 50% daily value |
| Calcium | 30% daily value |
| Magnesium | 10% daily value |
| Iron | 6% daily value |
| Phosphorous | 10% daily value |
| Folate | 15% daily value |

Table 19 compares Example J to the positive and positive control samples in order to evaluate the food product according to the anti-inflammatory standard defined by Index A and the anti inflammatory standard defined by Index B

**Table 19**

| | **Positive control** | **Example J** | **Negative control** | **Code** | **Index A** | **Index B** |
|---|---|---|---|---|---|---|
| iNOS | 0.37 ± 0.08 | 0.71 ± 0.06 | 1.00 ± 0.00 | Gold | -2 | -2 |
| COX-2 | 0.17 ± 0.10 | 2.97 ± 0.47 | 1.00 ± 0.00 | Purple | -4 | -4 |
| TNF-α | 244.8 ± 48.6 | 649.8 ± 17.6 | 953.7 ± 112.4 | Gold | -2 | -2 |
| NO | 7.4 ± 0.8 | 45.1 ± 0.6 | 43.0 ± 0.3 | Gold | -2 | n/a |
| PGE2 | 0.16 ± 0.1 | 41.66 ± 5.0 | 30.42 ± 1.5 | Gold | -4 | -4 |
| IL-6 | -1.05 ± 0.2 | 51.11 ± 1.0 | 43.51 ± 1.6 | Gold | 2 | n/a |
| IL-1β | 0.48 ± 0.1 | 20.05 ± 2.7 | 21.25 ± 1.2 | Gold | 2 | n/a |
| | | | | Total | -18 | -12 |

According to Index A, Example J was designated as pro Inflammatory According to Index B, Example J was designated as pro-inflammatory

One might expect that the high polyphenol composition of tart cherry juice, orange juice, and spiced almond milk would allow these beverages to produce the highest anti inflammatory responses. However, this expectation overlooks the important role of anti-inflammatory peptides produced following the digestion of soy proteins Thus, surprisingly, vanilla soymilk and soymilk combined with fruit and berry juices were especially beneficial in producing anti-inflammatory responses. The discovery that properly processed and formulated soymilk is effective as an anti inflammatory food provides rationale to include those soymilk products in diets to improve the health of people suffering from chronic inflammation. The evaluation method set forth above makes it feasible to compare the anti-inflammatory properties of whole, commercially processed foods and enables the creation of novel products based on an objective anti-inflammatory standard. Combinations of vegetables, fruits, seeds, herbs, spices, soymilk, yogurt cultures, and/or processing steps can be selected to improve anti-inflammatory responses.

The examples described in Table 1 were obtained during a study discussed in further detail below. The objective of the study was to evaluate the anti-inflammatory potential of pepsin-pancreatin hydrolysates of different commercially-available and prototype foods using lipopolysaccharide (LPS)-induced RAW 264.7 macrophages as an *in vitro* model. Eight different products were digested sequentially with pepsin and pancreatin to simulate gastrointestinal digestion and hydrolysates were freeze dried prior to evaluation of their anti-inflammatory property. RAW 264.7 macrophages were treated with 400 µg hydrolysates/mL and LPS (1 µg/mL) for 24 h. Hydrolysates from strawberry-banana soymilk ("SBH" - Example A above), mixed berry soymilk ("MXH" - Example B above) and vanilla soymilk ("SVMH" - Example C above) inhibited production of nitric oxide (27.9, 16.4 and 28.6%, respectively), interleukin-1β (26.3, 39.5 and 21.6%, respectively) and tumor necrosis factor-α (50.2, 47.5 and 33.3%, respectively). In addition, SBH, MXH and SVMH inhibited expressions of pro-inflammatory enzymes inducible nitric oxide synthase (66.7, 65.1 and 88.0%, respectively) and cyclooxygenase-2 (62.0, 69.9 and 40.6%, respectively). Antioxidant activity of the samples ranged from 32.4 to 319.7 µM Trolox equivalent. In conclusion, out of the eight samples evaluated vanilla, strawberry-banana and mixed berry soymilks can be used as potential anti-inflammatory products.

### Materials

Pepsin and pancreatin were purchased from Sigma-Aldrich (St. Louis, MO, USA). All commercially-available and prototype samples (pre-process soymilk, yogurt, soymilk with strawberry-banana juice and puree, soymilk with mixed berry juice, orange juice, pumpkin spice bread made to include soybean okara, tart cherry juice, soymilk with vanilla extract and almond milk with cinnamon) were provided by WhiteWave Foods (Broomfield, CO, USA), received in an enclosed-container, kept at 4 °C and analyzed within 24 h of arrival. Pumpkin bread was made from the following mixture: ¾ cup soybean okara, 2 ½ cup whole wheat flour, ½ cup cornmeal, 2 tsp baking soda, 1 tsp ground ginger, 1 ½ tsp ground cinnamon, ½ tsp ground nutmeg, ½ tsp ground cloves, 1/3 cup butter, 1/3 cup olive oil, 2 cup sugar, 2 cup cooked pumpkin mash, 4 eggs and 2/3 cup water. Murine macrophage RAW 264.7 cell line and Dulbecco's Modified Eagle Medium with L-glutamine (DMEM) were purchased from American Type Culture Collection (ATCC) (Manassas, VA, USA). Fetal bovine serum was from Invitrogen (Grand Island, NY, USA). CellTiter 96 Aqueous One Solution Proliferation assay kit (MTS/PES) was purchased from Promega Corporation (Madison, WI, USA). Mouse monoclonal antibodies [iNOS (sc-7271, epitope mapping between amino acids 1120-1145 near the C-terminus of iNOS of mouse origin), COX-2 (sc-19999, raised against a C-terminus peptide consisting of amino acids 580-598 of COX-2 of human origin), actin (sc-8432, specific for an epitope mapping between amino acids 350-375 at the C-terminus of actin of human origin)] were obtained from Santa Cruz Biotechnology (Santa Cruz, CA, USA) and antimouse IgG horseradish peroxidase conjugated secondary antibodies were from GE Healthcare (Buckinghamshire, UK). All other materials were purchased from Sigma-Aldrich, unless otherwise noted.

### Pepsin-pancreatin hydrolysis of samples

*In vitro* digestion of different samples was performed following previously reported protocol (Wang, W. Y., Bringe, N. A., Berhow, M. A., and de Mejia, E. G. Beta-conglycinins among sources of bioactives in hydrolysates of different soybean varieties that inhibit leukemia cells in vitro, Journal of Agricultural and Food Chemistry 56 (2008), pp. 4012-4020) with slight modifications. Briefly, 50-mL of liquid sample or 10 g of ground pumpkin bread suspended in 50-mL of deionized water was brought to 37 °C in a circulating water bath heater. Simulated gastrointestinal digestion was carried out by adding pepsin (800 to 2500 units/mg protein) at a ratio of 1:20 w/w (enzyme: protein content ratio) for 90 min at 37 °C pH 2 and pancreatin (8 x USP specifications) at the same enzyme: protein ratio for 90 min at 37 °C pH 7.5. After digestion, enzymes were inactivated by heating at 75 °C for 20 min. The resulting solution was centrifuged for 15 min at 27,000 x g, 4 °C. The supernatant was collected, desalted using 500 Da molecular weight cut-off membrane and lyophilized using FreeZone Freeze dry system (Kansas City, MO, USA). Sample hydrolysates were identified as follow: pre-process soymilk ("PPH" - Example I above), yogurt ("YH" - Example D above), soymilk with strawberry-banana juice and puree ("SBH" - Example A above), soymilk with mixed berry juice ("MXH" - Example B above), orange juice ("OJH" - Example G above), pumpkin bread made from soybean okara ("PBRH" - Example H above), tart cherry juice ("CJH" - Example E above), soymilk with vanilla extract ("SVMH" - Example C above), and almond milk with cinnamon ("AMH" - Example F above). Vanilla soymilk contained carrageenan. The mixed berry and strawberry-banana soymilks also contained apple and pear juices. The tart cherry juice contained apple juice. All lyophilized samples were stored at -20 °C prior to use in the cell culture study.

### Electrophoretic profile of different sample hydrolysates

Sample hydrolysates were mixed with Laemlli buffer with 5% β-mercaptoethanol and boiled for 5 min. The protein bands of hydrolysates were analyzed by SDS-PAGE using 4-20% Tris-HCl ready gels (Bio-Rad). A prestained precision Plus Protein standard (Bio-Rad) was used. The gel was run at 200 V for 35 min and washed with distilled water 3 times for 5 min each. After washing, gel was stained with Invitrogen SimplyBlue stain (Life Science Technologies, Grand Island, NY, USA) overnight. After destaining with water, the image of the gel was taken using GL 4000 Pro Imaging system (Carestream Health Inc., Rochester NY, USA).

### Soluble protein concentrations of different sample hydrolysates

Soluble protein concentration was determined using the DC protein assay by Bio-Rad (Hercules, CA, USA) following manufacturer's instructions. Briefly, 5 µl of diluted samples was plated in 96-well plate and 25 µL of reagent A was added. After which, 200 µL of reagent B was added, the plate was mixed gently and allowed to stand for 15 min at room temperature. The absorbance was read at 630 nm and protein concentration was calculated using the generated bovine serum albumin (BSA) standard curve (y = 0.00016 + 0.05066, R² = 0.993).

### Cell proliferation and treatment

Murine macrophage cell line RAW 264.7 was cultured in DMEM supplemented with 1% penicillin/streptomycin, 1% sodium pyruvate, and 10% fetal bovine serum at 37 °C in 5% CO₂/95% air using a CO2 Jacketed Incubator (NuAIRE DH Autoflow, Plymouth, MN, USA). The cell proliferation assay was conducted using the CellTiter 96® AQueous One Solution Proliferation assay kit containing a tetrazolium compound, 3-(4,5-dimethylthiazol-2-yl)-5-(3-carboxymethoxyphenyl)-2-(4-sulfophenyl)-2H-tetrazolium (MTS), and an electron coupling reagent, phenazine ethosulfate (PES) (Promega, Madison, WI). Briefly, 1x10⁴ cells were seeded in a 96-well plate and the total volume was adjusted to 200 µL with growth medium. After 24 h incubation, the cells were treated with 400 µg hydrolysates/mL (dissolved in PBS) and incubated for an additional 24 h. After this time, the growth medium was replaced with 100 µL fresh growth medium and 20 µL MTS/PES were added to each well. The plate was incubated for 2 h at 37 °C in 5% CO₂/95% air and the absorbance was read at 515 nm in an Ultra Microplate Reader (Biotek Instruments, Winooski, USA). The percentage of viable cells was calculated with respect to cells treated with PBS.

To test the anti-inflammatory potential of the hydrolysates, RAW 264.7 macrophages were seeded at 2x10⁵ in a six-well plate, and the total volume was adjusted to 2 mL with growth medium and incubated for 48 h at 37 °C in 5% CO₂/95% air. After 48 h incubation, the cells were treated with 400 µg hydrolysates/mL (dissolved in PBS), stimulated with 1 µg/mL lipopolysaccharide (LPS) and incubated for an additional 24 h. After 24 h, spent medium was collected for nitric oxide (NO), prostaglandin E₂ (PGE₂), TNF-α, IL-6 and IL-1β production measurement, and whole cell lysates were collected for western blot analysis of inflammatory markers, as follows. The concentration of 400 µg hydrolysates/mL was chosen based on previous studies that demonstrated that this concentration did not affect the viability of the macrophages.

### Nitric oxide measurement in supernatant of RAW 264.7 cell culture

Nitrite measurement was performed using Griess reaction. Briefly, 100 µL of the growth medium were plated in 96-well plate and an equal amount of Griess reagent (1% sulfanilamide and 0.1% N-1-(naphthyl)ethylenediamine-diHCl in 2.5% H3PO4) was added. The plate was incubated for 5 min at room temperature and the absorbance was measured at 550 nm in an Ultra Microplate Reader (Biotek Instruments, Winooski, USA). The amount of NO was calculated using the generated sodium nitrite standard curve (y = 0.1416x + 0.0861, R² = 0.997).

### Prostaglandin E₂ measurement in supernatant of RAW 264.7 cell culture

After 24 h of treatment and LPS induction, the culture supernatant was collected. PGE₂ was measured using a PGE₂ EIA monoclonal kit following the manufacturer's instructions (Cayman Chemical, Ann Arbor, MI). Briefly, 50 µL of diluted cell supernatant was plated in a 96-well goat antimouse IgG coated plate and incubated for 18 h at 4 °C. After incubation, the plate was washed using the provided washing buffer, and the color was developed by adding 200 µl of Ellman's reagent and shaking the plate for 60-90 min in the dark. The amount of PGE₂ was calculated using the generated PGE₂ standard curve (y = -2.1206x + 2.7587, R² = 0.991).

### TNF-α, IL-6 and IL-1β measurements in supernatant of RAW 264.7 cell culture

TNF-α, IL-6 and IL-1β were measured in cell culture supernatant using commercially available kit from Life Science Technologies. The procedure was done by following manufacturer's instructions for TNF-α (Cat # KMC3012), IL-6 (Cat # KMC0062) and IL-1β (Cat # KMC0012) measurements. The amount of TNF-α (y = 0.6102x + 1.7695, R2 = 0.980), IL-6 (y = 0.0015x + 0.0321, R2 = 0.992) and IL-1β (y = 0.0005x + 0.0467, R2 = 0.998) was calculated using their respective standard curves run at the same time as the treatments.

### Western blot analysis of iNOS and COX-2

Treated cells were washed twice with ice cold DMEM and twice with ice cold PBS and the cells were lysed with 200 µL of Laemmli buffer (Biorad) containing 5% β-mercaptoethanol. The cell lysates were sonicated for 30 s and then boiled for 5 min. Protein was quantified using the RCDC Assay (BioRad), and 25 µg protein was loaded in 4-20% Tris-HCl gels (BioRad) for protein separation. The resolved proteins were transferred to a PVDF membrane (Millipore, Billerica, MA) and blocked with 5% milk in 0.1% TBST for 1 h at 4 °C. After blocking, the membrane was washed with 0.1% TBST (5 times, 5 min each) and incubated with primary antibody (1:50) at 4°C overnight. The membrane was washed again and incubated with anti-mouse horseradish peroxidase conjugate secondary antibody (1:1000) for 2-3 h at room temperature. After incubation and repeated washing, the expression of proteins was visualized using chemiluminescent reagent (GE Healthcare) following manufacturer's instructions. The membrane picture was taken with a GL 4000 Pro Imaging system (Carestream Health Inc., Rochester NY). The intensity of the bands was quantified and normalized using actin from the same membrane stripped and probed with mouse anti-actin primary antibody.

### Antioxidant capacity of different pepsin-pancreatin hydrolysates

Antioxidant capacity was measured by the oxygen radical absorbance capacity (ORAC) assay as described previously (Prior, R., Hoang, H., Gu, L., Wu, X., Bacchiocca, M., Howard, L., Hampsch-Woodil, M., Huang, D. J., Ou, B. X., and Jacob, R. Assays for hydrophilic and lipophilic antioxidant capacity (oxygen radical absorbance capacity (ORAC(FL)) of plasma and other biological and food samples, Journal of Agricultural and Food Chemistry 51, (2003) pp. 3273-3279) using 20 µL trolox standard (1 - 160 µM), samples (400 µg hydrolysates/mL), or blank (75 mM phosphate buffer pH 7.4), 120 µL of 116.9 nM fluorescein (final concentration 70 nm/well), 60 µL of 40 mM AAPH per well. A black walled 96-well plate was read at 485 and 582 nm every 2 min at sensitivity 60 and 37 °C using a Synergy 2 multi-well plate reader (Biotek, Winooski, VT). Results were expressed as µmol Trolox equivalents (TE) and calculated using the generated Trolox standard curve (y = 0.1706x + 2.2325, R² = 0.990).

### Statistical Analysis

Statistical analysis was conducted using the proc GLM procedures of SAS version 9.3 (SAS Inst. Inc., Cary, NC). Group mean comparisons were conducted using LSD means and were considered to be significant at p < 0.05 based on minimum significant differences from one-way analysis of variance (ANOVA) with alpha = 0.05. All analyses were done in three independent replicates. Correlation between anti-inflammatory markers measured and antioxidant capacity was analyzed using Prism v 4.0 and statistical significance was reported at p < 0.05.

### Results

### Composition of different commercially-available and prototype foods

Tables 2, 4, 6, 8, 10, 13, 16, and 18 above show the composition of different commercially-available and prototype foods used in this study. Five of the samples were soy-based with protein content ranging from 5 g/240-mL for strawberry-banana soymilk and mixed berry soymilk to 6 g/240-mL for vanilla soymilk. Orange juice and tart cherry juice were used as control with protein content of 1 g/240-mL. Also, the amount of sugar per 240-mL varies from 5 g (for almond milk) to 28 g (for tart cherry juice) while the amount of fat ranges from 0 g (orange and tart cherry juices) to 3.5 g (vanilla soymilk ).

### Electrophoretic profile and soluble protein concentrations of pepsin-pancreatin hydrolysates from different commercially-available and prototype foods

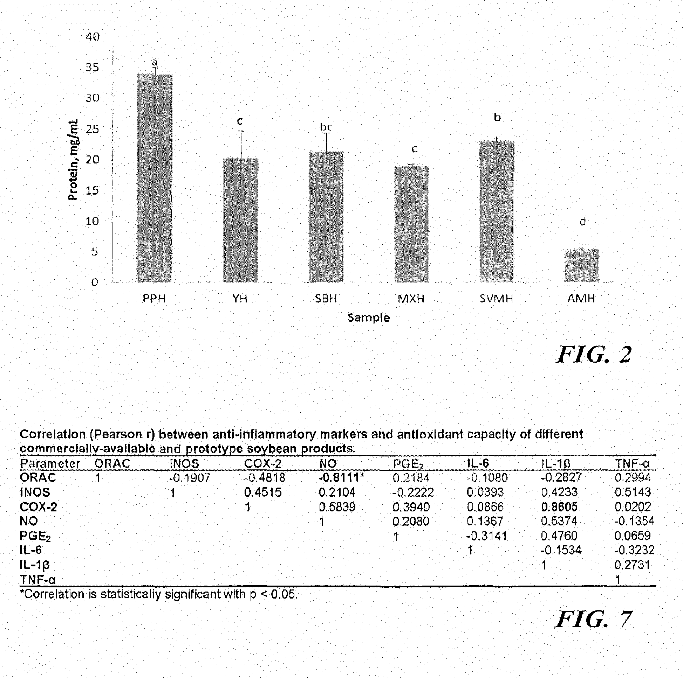

An SDS-PAGE profile of the pepsin-pancreatin hydrolysates of the different soy-based samples showed very similar protein profiles for PPH, YH, SBH, MXH, and SVMH, with most intense bands at the range of < 10 kDa to 20 kDa. On the other hand, OJH and PBRH showed weak band intensities with molecular masses < 10 kDa which can be attributed to very low protein concentrations present in these samples. FIG. 2 shows the soluble protein concentrations of the different pepsin-pancreatin hydrolysates ranging from 5 mg/mL (AMH) to 34 mg/mL (PPH).

### Effect of pepsin-pancreatin hydrolysates on NO/iNOS and PGE2/COX-2 pathway in LPS-induced RAW 264.7 macrophages

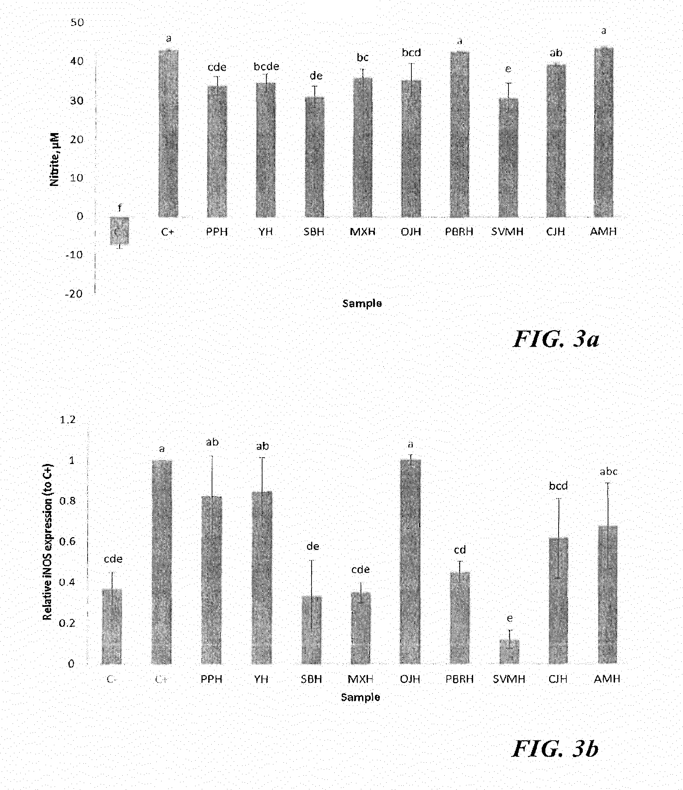

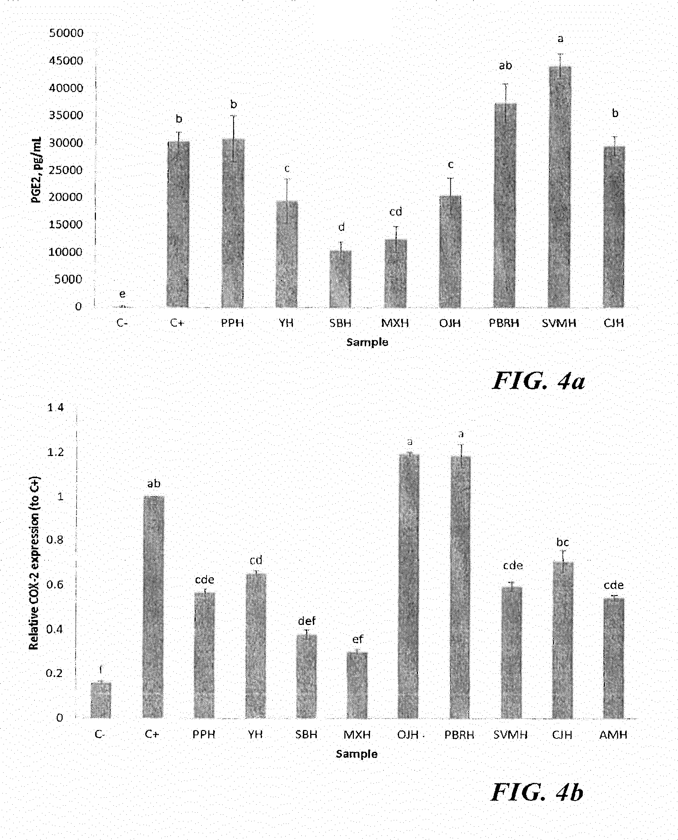

FIGURES 3 and 4 present the effect of pepsin-pancreatin hydrolysates from different products on NO (FIG. 3A)/iNOS (FIG. 3B) and PGE₂ (FIG. 4A)/COX-2 (FIG. 4B) pathway in LPS-stimulated macrophages. As shown in FIG. 3A, unstimulated macrophages (C-) did not produce any nitric oxide as determined by the sensitivity of Griess reagent and sodium nitrite standard curve used in this assay while LPS-stimulated macrophages (C+) produced NO at a level of 43 ± 0.3 (mean ± SEM) µM. Also, PPH, YH, SBH, MXH, OJH and SVMH treatments resulted in a statistically different reduction in the production of NO by LPS-stimulated macrophages. SBH and SVMH most potently inhibited NO production by 28.0 and 28.6%, respectively. FIG. 3B shows the effect of different hydrolysates on the expression of iNOS. As shown in this figure, SBH, MXH and SVMH most potently inhibited the expression of iNOS by 66.7, 65.1 and 88.0%, respectively. FIG. 4A shows the effect of different treatments on the production of PGE₂ by LPS-stimulated macrophages. YH, SBH and MXH treatments resulted in a statistically significant reduction in PGE₂ released by macrophages with SBH and MXH most potently inhibiting PGE₂ secretion by 65.8 and 59.0%, respectively. The observed reduction in the production of NO and PGE₂ can be explained by the capability of pepsin-pancreatin hydrolysates to inhibit the expression of enzymes responsible for their synthesis. In addition, SBH and MXH most potently inhibited COX-2 expression by 62.0 and 69.9%, respectively (FIG. 4B)

### Effect of pepsin-pancreatin hydrolysates on cytokine production of LPS-induced RAW 264.7 macrophages

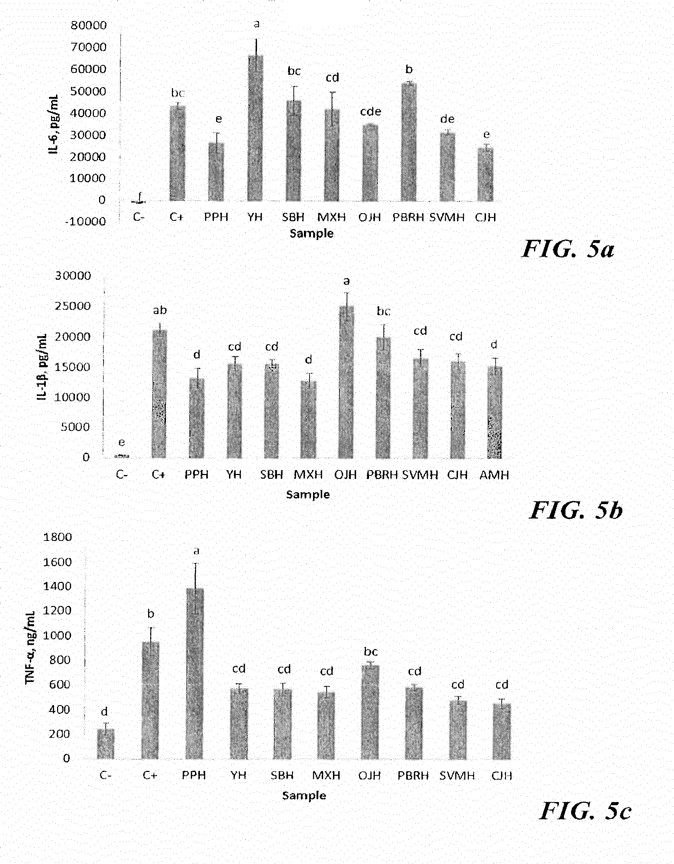

FIGURES 5A-5C show the effect of hydrolysates on the secretion of different cytokines (IL-6, IL-1β and TNF-α) of LPS-induced macrophages. As can be seen from FIG. 5A, IL-6 production was significantly reduced by PPH, SVMH and CJH by 38.9, 27.2 and 43.1%, respectively. IL-1β production was most potently inhibited by MXH treatment, leading to a 39.5% reduction (FIG. 5B). Treatment with YH, SBH, MXH, PBRH, SVMH and CJH resulted in significant reduction in TNF-α production by 39.9, 40.4, 42.8, 38.8, 49.8 and 52.4%, respectively as presented in FIG. 5C.

### Antioxidant activity of pepsin-pancreatin hydrolysates from different commercially-available and prototype foods

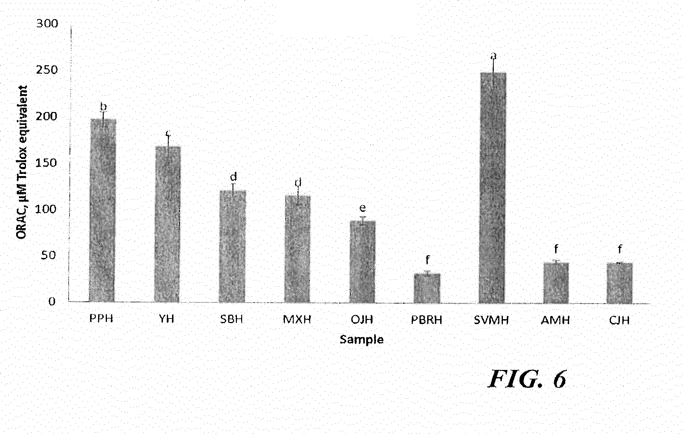

FIGURE 6 presents the antioxidant capacity of different hydrolysates as measured by ORAC. At 400 µg hydrolysates/mL, SVMH showed the highest antioxidant capacity of 249.1 µM TE while PBRH showed the lowest antioxidant capacity of 32.4 µM TE.

### Correlation between anti-inflammatory markers measured and antioxidant capacity

FIGURE 7 shows the correlation between the different anti-inflammatory markers measured as well as antioxidant capacity. Out of 28 pairings made, significant correlation (p < 0.05) was found only in 2 pairings namely ORAC and NO production and COX-2 expression and IL-1β secretion.

### Discussion

This study evaluated the potential anti-inflammatory property of different commercially-available and prototype food products using LPS-stimulated macrophages as an in vitro model. Pepsin-pancreatin hydrolysis was performed in order to mimic gastrointestinal digestion which is the major route of these products when ingested. Hydrolysis resulted in the conversion of large molecular mass soy proteins into smaller molecular peptides. It was found that hydrolysates from soymilk with vanilla (SVMH), strawberry-banana (SBH) and mixed berry (MXH) ingredients potently inhibited LPS-induced inflammation in vitro compared to hydrolysates from commercially available orange (OJH) and tart cherry juice (CJH). On the other hand, hydrolysates from spiced pumpkin bread containing soybean okara demonstrated weak anti-inflammatory potential.

These results suggest that the capability of these hydrolysates to possess anti-inflammatory property is dependent on the mixture of ingredients present in the products and how they are processed. For instance, vanilla soymilk was fortified with vitamins and minerals in addition to vanilla, and other soymilks contained added fruit juices. The addition of the fruit and berry components, in interaction with the peptides produced during simulated digestion, might have helped in the production of different biologically active components during hydrolysis with pepsin and pancreatin. It is also possible that peptides and fruit and berry polyphenols acted synergistically to inhibit an inflammation response. Comparing the anti-inflammatory property of pre-process soymilk and the final product yogurt showed that neither product potently inhibited LPS-induced inflammation in vitro suggesting that the microorganism used in the fermentation of the soymilk to produce yogurt did not impact the potential of soy to ameliorate inflammation.

SVMH, SBH and MXH potently inhibited pro-inflammatory markers such as NO, iNOS expression, COX-2 expression and the release of cytokines IL-1β and TNF-α. Uncontrolled and aberrant production of these pro-inflammatory markers is associated with different diseases and metabolic disorders such as obesity, cardiovascular disease and cancer. It is then important to have food products that can be consumed on a daily basis that can prevent uncontrolled production of TNF-α during inflammatory states to prevent the initiation and progression of inflammatory-related diseases. IL-1β was also downregulated by SVMH, SBH and MXH treatments which can also suggest the protective effects of these hydrolysates against inflammatory-related cardiovascular disease. The reduction of IL-1β secreted by macrophages may explain the reduced expression of pro-inflammatory enzymes COX-2 and iNOS observed from SVMH, SBH and MXH treated macrophages (IL-1β can induce expression of these pro-inflammatory enzymes in endothelial cells, see Dinarello C. A. Immunological and inflammatory functions of the interleukin-1 family, Annual Review of Immunology 27 (2009), pp. 519-550)). In addition, a correlation was observed between the following pro-inflammatory markers: COX-2, IL-1β, ORAC, and NO.

In conclusion, it was found that pepsin-pancreatin hydrolysates of different commercially-available and prototype products have different potential to reduce LPS-stimulated inflammation in vitro. Out of the products tested, SVMH, SBH and MXH showed the most potent inhibitory effects on the different pro-inflammatory markers measured suggesting that these products should be studied in human trials to further confirm anti-inflammatory effects.

In certain embodiments, a computing system may be used to facilitate evaluating the food product according to an anti-inflammatory standard. The computing system may include an interface, logic, memory, and/or other suitable element. The interface receives input, sends output, processes the input and/or output, and/or performs other suitable operation. In certain embodiments, the interface receives data indicating the expression level of the selected markers and outputs a result of applying the anti-inflammatory standard to a display screen, a printer, or other suitable output. The interface may comprise hardware and/or software.

The logic performs the operations of the component, for example, executes instructions to generate output from input. In certain embodiments, the logic may apply the anti-inflammatory standard to the data indicating the expression level of the selected markers. The logic may include hardware (e.g., one or more processors), software, (e.g., one or more applications), and/or other logic. The logic may be encoded in one or more tangible, non-transistory media and may perform operations when executed by a computer. Certain logic, such as a processor, may manage the operation of a component. Examples of a processor include one or more computers, one or more microprocessors, one or more applications, and/or other logic.

In particular embodiments, the operations of the embodiments may be performed by one or more computer readable media encoded with a computer program, software, computer executable instructions, and/or instructions capable of being executed by a computer. In particular embodiments, the operations of the embodiments may be performed by one or more computer readable media storing, embodied with, and/or encoded with a computer program and/or having a stored and/or an encoded computer program.

Memory stores information. The memory may comprise one or more tangible, computer-readable, and/or computer-executable storage medium, and may exclude signals or carrier waves. Examples of memory include computer memory (for example, Random Access Memory (RAM) or Read Only Memory (ROM)), mass storage media (for example, a hard disk), removable storage media (for example, a Compact Disk (CD) or a Digital Video Disk (DVD)), database and/or network storage (for example, a server), and/or other computer-readable medium.

Although the present disclosure has been described with several embodiments, numerous changes, variations, alterations, transformations, and modifications can be suggested to one skilled in the art, and it is intended that the present disclosure encompass such changes, variations, alterations, transformations, and modifications as fall within the scope of the appended claims. For example, modifications, additions, or omissions can be made to systems disclosed herein without departing from the scope of the invention. The components can be integrated or separated. Moreover, the operations can be performed by more, fewer, or other components. Similarly, modifications may be made to the methods disclosed herein without departing from the scope of the invention. The steps can be combined, modified, or deleted where appropriate, and additional steps can also be added to those shown. Additionally, the steps can be performed in any suitable order. Although the preceding examples describe certain embodiments of the disclosure, any suitable ingredients may be added, modified, or deleted without departing from the scope of the present disclosure.

The present invention relates to the following embodiments:
1. A method comprising:
   selecting one or more inflammatory markers to evaluate, the one or more inflammatory markers selected from the group consisting of iNOS, COX-2, TNF-alpha, NO, PGE₂, IL-6, and IL-1β;
   determining a first expression level of the one or more inflammatory markers, the first expression level associated with a positive control sample comprising a type of human macrophages;
   determining a second expression level of the one or more inflammatory markers, the second expression level associated with a test sample obtained by exposing a food product to a digest, the type of human macrophages, and a pro-inflammatory compound;
   performing a comparison between the first expression level and the second expression level; and
   designating the food product according to an anti-inflammatory standard based on the comparison.
2. The method of Embodiment 1, further comprising:
   determining a third expression level of the one or more inflammatory markers, the third expression level associated with a negative control sample comprising the type of human macrophages and the pro-inflammatory compound;
   wherein performing the comparison further comprises comparing the second expression level and the third expression level.
3. The method of Embodiment 2, wherein the anti-inflammatory standard scores each of the one or more inflammatory markers such that:
   the first expression level corresponds to a good score;
   the third expression level corresponds to a bad score; and
   the second expression level is scored relative to the first expression level and the third expression level.
4. The method of any of Embodiments 1 to 3, wherein designating the food product according to an anti-inflammatory standard comprises designating the food product as anti-inflammatory if the second expression level is statistically not significantly different from the first expression level.
5. The method of any of Embodiments 1 to 4, further comprising:
   determining that the second expression level is not significantly different from the first expression level if the p value is less than 0.05.
6. The method of any of Embodiments 1 to 5, wherein the food product comprises a non-dairy milk.
7. The method of any of Embodiments 1 to 6, wherein the selected inflammatory markers include iNOS, COX-2, TNF-alpha, and PGE₂.
8. The method of any of Embodiments 1 to 7, wherein the digest comprises pepsin or pancreatin.
9. The method of any of Embodiments 1 to 8, wherein:
   the food product is exposed to the digest and the type of human macrophages for between approximately 1 to 3 hours prior to exposing the food product to the pro-inflammatory compound; and
   the second expression level is determined a pre-determined amount of time after exposing the food product to the pro-inflammatory compound, the pre-determined amount of time between approximately 20 and 30 hours.
10. The method of Embodiment 9, further comprising:
   determining a third expression level of the one or more inflammatory markers, the third expression level associated with a negative control sample comprising the type of human macrophages exposed to the pro-inflammatory compound for approximately the same pre-determined amount of time between approximately 20 and 30 hours;
   wherein performing the comparison further comprises comparing the second expression level and the third expression level.
11. A method comprising:
   selecting one or more inflammatory markers to evaluate, the one or more inflammatory markers selected from the group consisting of iNOS, COX-2, TNF-alpha, NO, PGE₂, IL-6, and IL-1β;
   determining an expression level of the one or more inflammatory markers associated with a test sample, the test sample comprising a food product exposed to a digest, a type of human macrophages, and a pro-inflammatory compound; and
   designating the food product according to an anti-inflammatory standard based on the expression level of the one or more inflammatory markers.
12. The method of Embodiment 11, further comprising obtaining a negative control sample comprising the type of human macrophages and the pro-inflammatory compound; and
   determining a second expression level of the one or more inflammatory markers, the second expression level associated with the negative control sample;
   wherein the designating the food product according to an anti-inflammatory standard is based on comparing the expression level and the second expression level.
13. The method of Embodiment 11 or 12, the anti-inflammatory standard designates the food product as anti-inflammatory if the expression level for each of the selected inflammatory markers falls statistically below a corresponding predetermined threshold, the predetermined threshold indicating that the test sample expresses the selected inflammatory marker at a sufficiently lower level than the level at which the negative control sample expresses the selected inflammatory marker.
14. The method of any of Embodiments 11 to 13, wherein:
   the predetermined threshold for iNOS is at least a 65% reduction compared to the second expression level;
   the predetermined threshold for COX-2 is at least a 62% reduction compared to the second expression level;
   the predetermined threshold for TNF-alpha is at least a 40% reduction compared to the second expression level;
   the predetermined threshold for NO is at least a 19% reduction compared to the second expression level;
   the predetermined threshold for PGE₂ is at least a 66% reduction compared to the second expression level;
   the predetermined threshold for IL-6 is at least a 24% reduction compared to the second expression level; and
   the predetermined threshold for IL-1β is at least a 25% reduction compared to the second expression level.
15. The method of any of Embodiments 11 to 14, wherein:
   the food product comprises soymilk and one or more of: a vegetable ingredient, a fruit ingredient, a seed, an herb, a spice, a plant-based protein ingredient, and a yogurt culture; and
   the food product has been pasteurized or homogenized.
16. The method of any of Embodiments 11 to 15, wherein the selected inflammatory markers include iNOS, COX-2, TNF-alpha, and PGE₂.
17. A beverage comprising:
   at least one gram of protein per eight ounce serving;
   a healthy bioactivity index greater than 5;
   wherein the beverage is designated as anti-inflammatory.
18. The beverage of Embodiment 17, wherein the beverage is plant-based.
19. The beverage of Embodiment 17, wherein the beverage comprises soymilk and one or more of: a vegetable ingredient, a fruit ingredient, a plant-based protein ingredient, a seed, an herb, a spice, and a yogurt culture; and
   the beverage has been pasteurized or homogenized.
20. The beverage of any of Embodiments 17 to 19, wherein the healthy bioactivity index corresponds to Index A or Index B.

## Claims

1. A method comprising:
selecting one or more inflammatory markers to evaluate, the one or more inflammatory markers selected from the group consisting of iNOS, COX-2, TNF-alpha, NO, PGE₂, IL-6, and IL-1β;
determining a first expression level of the one or more inflammatory markers, the first expression level associated with a positive control sample comprising a type of human macrophages;
determining a second expression level of the one or more inflammatory markers, the second expression level associated with a test sample obtained by exposing a food product to a digest, the type of human macrophages, and a pro-inflammatory compound;
performing a comparison between the first expression level and the second expression level; and
designating the food product according to an anti-inflammatory standard based on the comparison.

2. The method of Claim 1, further comprising:
determining a third expression level of the one or more inflammatory markers, the third expression level associated with a negative control sample comprising the type of human macrophages and the pro-inflammatory compound;
wherein performing the comparison further comprises comparing the second expression level and the third expression level.

3. The method of Claim 2, wherein the anti-inflammatory standard scores each of the one or more inflammatory markers such that:
the first expression level corresponds to a good score;
the third expression level corresponds to a bad score; and
the second expression level is scored relative to the first expression level and the third expression level.

4. The method of any of Claims 1 to 3, wherein designating the food product according to an anti-inflammatory standard comprises designating the food product as anti-inflammatory if the second expression level is statistically not significantly different from the first expression level.

5. The method of any of Claims 1 to 4, wherein the food product comprises a non-dairy milk.

6. The method of any of Claims 1 to 5, wherein the selected inflammatory markers include iNOS, COX-2, TNF-alpha, and PGE₂.

7. The method of any of Claims 1 to 6, wherein the digest comprises pepsin or pancreatin.

8. The method of any of Claims 1 to 7, wherein:
the food product is exposed to the digest and the type of human macrophages for between approximately 1 to 3 hours prior to exposing the food product to the pro-inflammatory compound; and
the second expression level is determined a pre-determined amount of time after exposing the food product to the pro-inflammatory compound, the pre-determined amount of time between approximately 20 and 30 hours.

9. The method of Claim 8, further comprising:
determining a third expression level of the one or more inflammatory markers, the third expression level associated with a negative control sample comprising the type of human macrophages exposed to the pro-inflammatory compound for approximately the same pre-determined amount of time between approximately 20 and 30 hours;
wherein performing the comparison further comprises comparing the second expression level and the third expression level.

10. A method comprising:
selecting one or more inflammatory markers to evaluate, the one or more inflammatory markers selected from the group consisting of iNOS, COX-2, TNF-alpha, NO, PGE₂, IL-6, and IL-1β;
determining an expression level of the one or more inflammatory markers associated with a test sample, the test sample comprising a food product exposed to a digest, a type of human macrophages, and a pro-inflammatory compound; and
designating the food product according to an anti-inflammatory standard based on the expression level of the one or more inflammatory markers.

11. The method of Claim 10, further comprising obtaining a negative control sample comprising the type of human macrophages and the pro-inflammatory compound; and
determining a second expression level of the one or more inflammatory markers, the second expression level associated with the negative control sample;
wherein the designating the food product according to an anti-inflammatory standard is based on comparing the expression level and the second expression level.

12. The method of Claim 10 or 11, the anti-inflammatory standard designates the food product as anti-inflammatory if the expression level for each of the selected inflammatory markers falls statistically below a corresponding predetermined threshold, the predetermined threshold indicating that the test sample expresses the selected inflammatory marker at a sufficiently lower level than the level at which the negative control sample expresses the selected inflammatory marker.

13. The method of any of Claims 10 to 12, wherein:
the food product comprises soymilk and one or more of: a vegetable ingredient, a fruit ingredient, a seed, an herb, a spice, a plant-based protein ingredient, and a yogurt culture; and
the food product has been pasteurized or homogenized.

14. The method of any of Claims 10 to 13, wherein the selected inflammatory markers include iNOS, COX-2, TNF-alpha, and PGE₂.

15. A beverage comprising:
at least one gram of protein per eight ounce serving;
a healthy bioactivity index greater than 5;
wherein the beverage is designated as anti-inflammatory.
